# EUROPEAN PATENT SPECIFICATION

(11) **EP 4 479 004 B1**
(45) Date of publication and mention of the grant of the patent: **14.10.2026**
(21) Application number: 23703343.6
(22) Date of filing: 27.01.2023
(51) Int. Cl.: A61F 9/007, A61M 1/00

(54) **CONTROLLING CONSTANT INTRAOCULAR PRESSURE USING ADAPTIVE FLOW RATES OF IRRIGATION AND ASPIRATION FLUIDS**
STEUERUNG DES KONSTANTEN INTRAOKULAREN DRUCKS MITTELS ADAPTIVER DURCHFLUSSRATEN VON SPÜL- UND ASPIRATIONSFLÜSSIGKEITEN
CONTRÔLE DE LA PRESSION INTRAOCULAIRE CONSTANTE À L'AIDE DE DÉBITS ADAPTATIFS DE FLUIDES D'IRRIGATION ET D'ASPIRATION

(30) Priority: 14.02.2022 US 202217670783
(43) Date of publication of application: 25.12.2024
(73) Proprietor: Johnson & Johnson Surgical Vision, Inc., Irvine, CA 92618 (US)
(72) Inventor: GLINER, Vadim, 2066717 Yokneam (IL); GOVARI, Assaf, 2066717 Yokneam (IL)
(74) Representative: Carpmaels & Ransford LLP
(86) International application number: PCT/IB2023/050734
(87) International publication number: WO 2023/152591

(56) References cited:
- WO-A1-2021/001742
- US-A1- 2018 256 395
- US-A1- 2019 099 547
- US-A1- 2021 146 019

## Description

### FIELD OF THE DISCLOSURE

The present disclosure relates generally to phacoemulsification systems and probes, and particularly to controlling intraocular pressure in phacoemulsification procedures.

### BACKGROUND OF THE DISCLOSURE

A cataract is a clouding and hardening of the eye's natural lens, a structure which is positioned behind the cornea, iris and pupil. The lens is mostly made up of water and protein and as people age these proteins change and may begin to clump together obscuring portions of the lens. To correct this, a physician may recommend phacoemulsification cataract surgery. In the procedure, the surgeon makes a small incision in the sclera or cornea of the eye. Then a portion of the anterior surface of the lens capsule is removed to gain access to the cataract. The surgeon then uses a phacoemulsification probe, which has an ultrasonic handpiece with a needle. The tip of the needle vibrates at ultrasonic frequency to break and emulsify the cataract while a pump aspirates particles and fluid from the eye through the tip. Aspirated fluids are replaced with irrigation of a balanced salt solution to maintain constant intraocular pressure (IOP) in the anterior chamber of the eye. The cataract removal must be carried out while retaining constant IOP in order to prevent permanent damage to the eye. US 2021/0146019 Al describes a medical instrument such as a phacoemulsification probe which comprises a shared vane pump and an auxiliary vane pump.

After removing the cataract with phacoemulsification, the softer outer lens cortex is removed with suction. An intraocular lens (IOL) is then introduced into the empty lens capsule restoring the patient's vision.

### SUMMARY OF THE INVENTION

The invention provides a phacoemulsification system in accordance with appended claim 1, and a calibration method in accordance with appended claim 8. Certain embodiments are defined in the dependent claims. Where methods of surgery are described in the present disclosure, these do not form part of the claimed invention, but are provided for understanding the invention as claimed. The present disclosure will be more fully understood from the following detailed description of the examples thereof, taken together with the drawings in which:

### BRIEF DESCRIPTION OF THE DRAWINGS

Fig. 1 is a schematic, pictorial illustration of a cataract surgery carried out using a phacoemulsification system, in accordance with an example of the present disclosure; and
Fig. 2 is a schematic, pictorial illustration of a calibration chamber fitted over a needle and a sleeve of the phacoemulsification probe, in accordance with an example of the present disclosure.
Fig. 3 is a flow chart that schematically illustrates a method for controlling constant intraocular pressure using adaptive flow rates of irrigation and aspiration fluids, in accordance with an example of the present disclosure.

### DETAILED DESCRIPTION OF EXAMPLES

### OVERVIEW

During phacoemulsification of an eye lens, emulsified lens particles are aspirated from the eye, while applying irrigation fluid to the eye. It is important to match between the amount of irrigation fluid applied to the eye and emulsified lens particles and irrigation fluid aspirated from the eye, so as to maintain a constant intraocular pressure (IOP) in the eye.

Examples of the present disclosure that are described hereinafter provide techniques for controlling constant IOP during a cataract surgery, using adaptive flow rates of the irrigation and aspiration fluids.

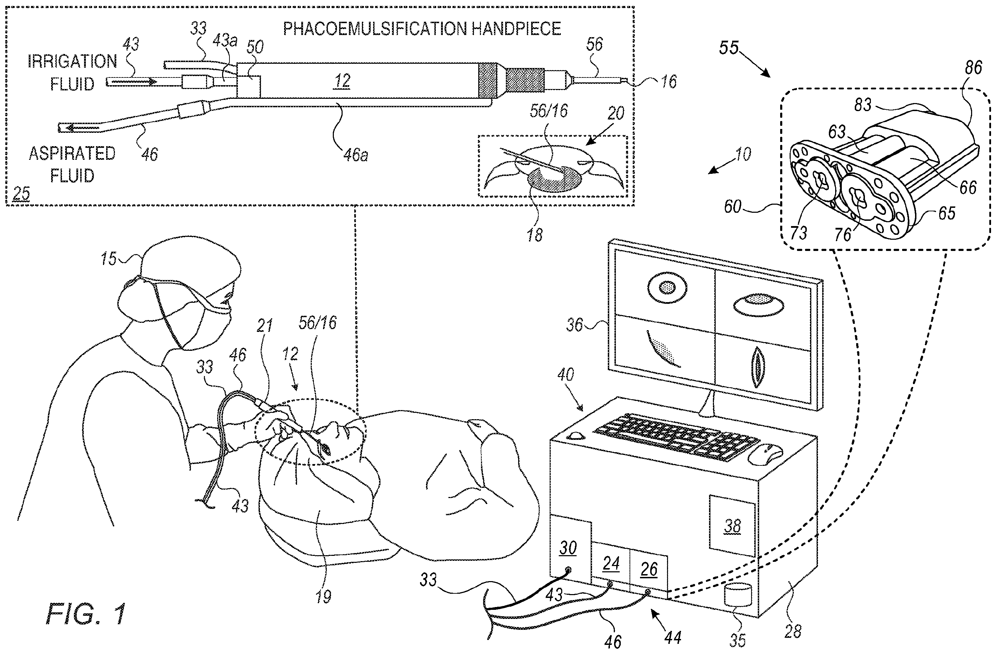

In some examples, a phacoemulsification system comprises an ultrasonic handpiece having a needle mounted thereon. The tip of the needle is configured to vibrate at an ultrasonic frequency to break the cataract into particles, which are emulsified in fluids applied to the eye. The system comprises two progressive cavity pumps (PCPs) integrated together in a pumping assembly, which is referred to herein as a pump having one or two electric motors, and is described in detail in Fig. 1 below.

In some examples, the pump is configured to aspirate the emulsified particles and fluid, also referred to herein as aspiration fluid(s), from the treated eye while applying irrigation fluid to the eye, so as to maintain constant IOP in the eye. The irrigation and aspiration fluids flow between the pump and the eye through the tip of the needle and sleeve.

In some examples, the pump comprises a cassette having irrigation and aspiration chambers configured to flow the irrigation and aspiration fluids therethrough, respectively. In principle, the irrigation and aspiration chambers of the cassette are designed to have a similar volume. In some cases, however, the volume of the chambers differs from one another (e.g., by up to 5%) due to variations in the production process of the cassette, also referred to herein as manufacturing tolerances. In such cases, the flow rate of the irrigation and/or aspiration fluids must be adjusted to compensate for the difference between the volume of the irrigation and aspiration chambers. Note that the flow rate adjustments are essential for matching the volume of irrigation and aspiration fluids exchanged between the pump and the eye in question.

A possible solution to the problem of unequal volume of the cassette chambers is to measure the pressure in both irrigation and aspiration lines, and responsively, to adjust the flow rates of the irrigation and aspiration fluids to obtain a target constant pressure in both irrigation and aspiration lines. In the present example the flow rate is altered by adjusting the operating speed (e.g., revolutions per minute) of the motor(s) of the pump. This solution, however, is prone to aggressive operation of the system in response to various situations that may occur during the cataract surgery. For example, a sudden drop in vacuum may be caused in response to an occluding particle at the tip of needle being emulsified and aspirated.

In some examples, the phacoemulsification system may be operated using constant flows (rather than constant pressure) of the irrigation and aspiration fluids, after performing a calibration procedure described herein.

In some examples, the calibration procedure begins with coupling, to a distal end of a phacoemulsification handpiece, a needle and sleeve having a given diameter, and fitting an expandable calibration chamber on a tip of the needle and sleeve. The calibration chamber is configured to expand (e.g., inflate) and collapse (e.g., deflate) based on the amount of fluid residing therein. More specifically, when the inflowing volume of fluid is larger than the outflowing volume, the calibration chamber is configured to inflate, and when the outflowing volume is larger than the inflowing volume of fluid, the calibration chamber is configured to deflate. Subsequently, a user operates the pump associated with the irrigation line coupled with the phacoemulsification handpiece at a target constant flow rate and a processor of the system records the irrigation flow rate sensed by a suitable sensing device during a predefined time interval. At the same time, the user operates the pump associated with the aspiration line coupled with the phacoemulsification handpiece to obtain a constant target pressure of the fluid residing in the calibration chamber. Moreover, flow rate and pressure of the aspiration fluid are measured in the aspiration line, and the readings thereof are recorded by the processor during the same predefined time interval.

In some examples, the processor is configured to compute integrals of the irrigation and aspiration flow rates recorded during the predefined time interval, and to calculate a ratio of flow rates required to obtain constant pressure in the calibration chamber. Note that the pressure in the calibration chamber is indicative of the IOP in the eye during a cataract surgery.

In some examples, the user may repeat the calibration process described above with a different type of a needle and sleeve, and/or a different cassette and/or using different parts and operational conditions of irrigation and aspiration. Moreover, the processor is configured to produce or use a lookup table (LUT) comprising the calculated flow rate ratio for each of the aforementioned parts and/or operational condition.

In some examples, in setting up the cataract surgery the user selects a needle, a cassette of the pump and other parts, and sets the operational conditions of the surgery. In such examples, the processor applies a suitable flow rate ratio of the LUT, so as to set respective constant flow rates of the irrigation and aspiration fluids for obtaining constant intraocular pressure. Subsequently, the user begins the cataract surgical procedure, and based on the ratios in the LUT, the processor controls the pump to execute the respective constant flow rates of the irrigation and aspiration fluids.

The disclosed technique improves the safety of phacoemulsification procedures, for example, by compensating for manufacturing tolerances in the irrigation and aspiration chambers of the cassette of the respective progressive cavity pumps.

### SYSTEM DESCRIPTION

Fig. 1 is a schematic, pictorial illustration of a cataract surgery carried out using a phacoemulsification system 10, in accordance with an example of the present disclosure.

In some examples, phacoemulsification system 10 comprises a phacoemulsification probe 12, also referred to herein as a phacoemulsification handpiece or as a probe 12, for brevity, shown in an inset 25. Phacoemulsification probe 12 comprises a needle 16 and a coaxial irrigation sleeve 56 that at least partially surrounds needle 16 and creates a fluid pathway between the external wall of the needle and the internal wall of irrigation sleeve 56. Note that needle 16 is hollow, has a predefined diameter at the tip, and is configured to function as an aspiration channel. Moreover, irrigation sleeve may have one or more side ports at or near the distal end to allow irrigation fluid to flow towards the distal end of the handpiece through the fluid pathway and out of the port(s).

In some examples, needle 16 is configured for insertion into a lens capsule 18 of an eye 20 of a patient 19 by a physician 15 to remove a cataract. While needle 16 (and irrigation sleeve 56) are shown in inset 25 as a straight object, any suitable needle may be used with phacoemulsification probe 12, for example, a curved or bent tip needle commercially available from Johnson & Johnson Surgical Vision, Inc., Irvine, CA, USA.

Reference is now made to the general view of Fig. 1. In an example, system 10 comprises a console 28 having a pump 44 comprising: (i) a pumping sub-system 24 for irrigation, (ii) a pumping sub-system 26 for aspiration, and (iii) a cassette 55, which is shown in an inset 60, and is described in detail below. During the phacoemulsification procedure, pump 44 is configured to pump irrigation fluid from an irrigation reservoir (not shown) located within or coupled externally with pumping sub-system 24, and to flow the irrigation fluid to irrigation sleeve 56 for irrigating eye 20 of patient 19. The irrigation fluid is pumped via an irrigation (tubing) line 43 running from console 28 to an irrigation channel 43a of probe 12.

Reference is now made back to inset 25. In an example, aspiration fluid(s), such as eye fluid and waste matter (e.g., emulsified parts of the cataract), are aspirated from eye 20 by pump 44. The aspiration fluids are aspirated through hollow needle 16, via an aspiration channel 46a of probe 12 and an aspiration (tubing) line 46, to the collection receptacle (also referred to herein as a waste reservoir) (not shown) located within or coupled externally to pumping sub-system 26.

Reference is now made to inset 60. In some examples, cassette 55 comprises two progressive cavity pumps (PCPs) configured to be inserted into and removed from console 28, and to actuate each of the irrigation and aspiration flows. Each of the PCPs comprises a stator and a rotor, which couple with a motor of pump 44 and together are configured to move the fluids into and out of the eye. In the present example, cassette 55 comprises an aspiration chamber 66, which is made from a hollow tube. The volume of aspiration chamber 66 is defined by: (i) the inner diameter of the hollow tube, and (ii) the distance between a rotatable screw 86 and an opening 76, which is configured to couple with aspiration line 46. Similarly, cassette 55 comprises an irrigation chamber 63, which is also made from a hollow tube. The volume of irrigation chamber 63 is defined by: (i) the inner diameter of the hollow tube, and (ii) the distance between a rotatable screw 83 and an opening 73, which is configured to couple with irrigation line 43. In the present example, rotatable screw 83 is configured to be rotatably actuated by the respective motor of the PCP for flowing the irrigation fluid, and rotatable screw 86 is configured to be rotatably actuated by the respective motor of the PCP for flowing the aspiration fluid.

In an example, cassette 55 comprises a bracket 65, which is configured to serve as a handle for inserting and removing cassette 55 into and out of console 28, respectively. In an inserted position, chambers 63 and 66 and rotatable screws 83 and 86 are being inserted into console 28, whereas bracket 65 and openings 73 and 76 are positioned on an external panel of console 28 and are visible to a user of system 10. In this configuration, the user of system 10 may connect or disconnect between irrigation line 43 and opening 73, and between aspiration line 46 and openings 76.

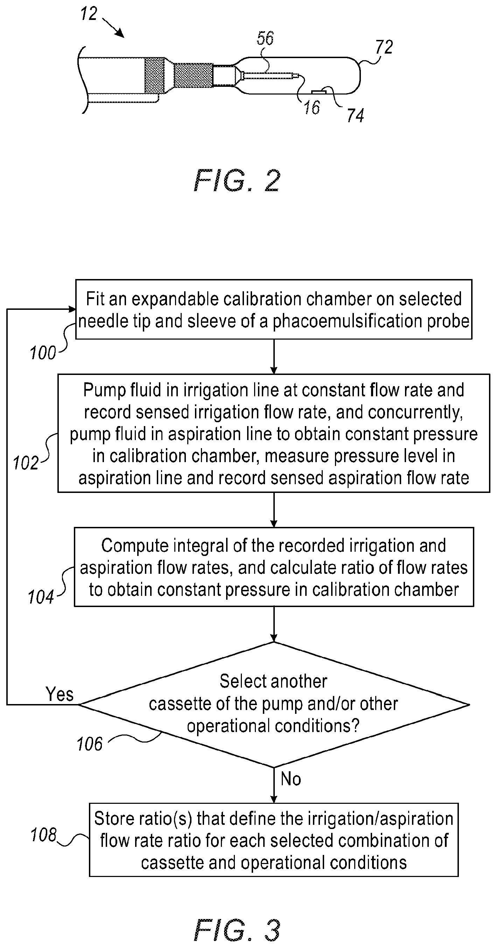

In some cases, the volume of irrigation chamber 63 and aspiration chamber 66 may differ from one another, typically due to process variations that may occur during the production of cassette 55. The volume difference may be caused, for example, by uneven inner diameter of the respective hollow tubes, and/or by uneven distance between the respective opening and the rotatable screws of the respective chambers. Note that an equal flow rate of the irrigation and aspiration fluids, together with a difference in the volume of chambers 63 and 66, typically results in a different volume of irrigation and aspiration fluids flowing into and out of eye 20, respectively. Such differences typically alter the IOP within eye 20, and may cause a damage to the eye. A method for maintaining an equal volume of irrigation and aspiration fluids flowing into and out of eye 20, respectively, is described in detail in Fig. 3 below.

In another example, the pumping sub-system 24 may be coupled or replaced with a gravity fed irrigation source such as a balanced salt solution bottle/bag.

In some examples, phacoemulsification probe 12 may comprise additional elements (not shown), such as a piezoelectric crystal coupled to a horn to drive vibration of needle 16. The piezoelectric crystal is configured to vibrate needle 16 in a resonant vibration mode. The vibration of needle 16 is used to break a cataract of eye 20 into small pieces during a phacoemulsification procedure. Console 28 comprises a piezoelectric drive module 30, coupled with the piezoelectric crystal, using electrical wiring running in a cable 33. Drive module 30 is controlled by a processor 38 and conveys processor-controlled driving signals via cable 33, for example, to maintain needle 16 at maximal vibration amplitude. The drive module may be implemented in hardware or software, for example, in a proportional-integral-derivative (PID) control architecture.

In some embodiments, processor 38 may receive user-based commands via a user interface 40, which may comprise setting a vibration mode and/or frequency of the piezoelectric crystal, and setting or adjusting an irrigation and/or aspiration flow rate obtained by controlling pump 44. In an example, user interface 40 and display 36 may be combined as a single touch screen graphical user interface. In an example, physician 15 may use a foot pedal (not shown) as a means of control. Additionally, or alternatively, processor 38 may receive the user-based commands from controls located in a handle 21 of probe 12.

In an example, some or all of the functions of processor 38 may be combined in a single physical component or, alternatively, implemented using multiple physical components. These physical components may comprise hard-wired or programmable devices, or a combination of the two. In some examples, at least some of the functions of processor 38 may be carried out using suitable software stored in a memory 35 implemented within console 28. This software may be downloaded to a device in an electronic form, over a network, for example. Alternatively, or additionally, the software may be stored in tangible, non-transitory computer-readable storage media, such as optical, magnetic, or electronic memory.

System 10 of Fig. 1 may comprise further elements which are omitted for clarity of presentation. For example, physician 15 typically performs the procedure using a stereomicroscope or magnifying glasses, neither of which are shown. Physician 15 may use other surgical tools in addition to or instead of probe 12, which are also not shown in order to maintain clarity and simplicity of presentation.

### USING ADAPTIVE FLOW RATES TO COMPENSATE FOR VOLUME DIFFERENCE IN IRRIGATION AND ASPIRATION CHAMBERS

Fig. 2 is a schematic, pictorial illustration of a calibration chamber 72 fitted over needle 16 and sleeve 56 of phacoemulsification probe 12, in accordance with an example of the present disclosure.

In some examples, calibration chamber 72 resembles eye 20, and is used in a calibration procedure described in Fig. 3 below. In some examples, calibration chamber 72 comprises any suitable type of an expandable member, and is configured to exchange fluids with phacoemulsification probe 12, via needle 16 and sleeve 56. When the volume of fluid flowing into calibration chamber 72 is larger than the volume of outflowing fluids, calibration chamber 72 is configured to inflate. When the outflowing volume is larger than the inflowing volume of fluid, calibration chamber 72 is configured to deflate. The inflation and deflation of calibration chamber 72 are indicative of change in the static pressure of the fluids in calibration chamber 72.

In some examples, one or more sensors 74, such as pressure and/or flow sensors, are located within calibration chamber 72 for sensing the pressure and/or flow rate of the fluids. Additionally, or alternatively, sensors 74 may be located at one or more suitable positions within phacoemulsification probe 12, anywhere along irrigation line 43 and aspiration line 46 shown in Fig. 1 above and/or in console 28.

Fig. 3 is a flow chart that schematically illustrates a method for controlling constant intraocular pressure (IOP) in eye 20 using adaptive flow rates of irrigation and aspiration fluids, in accordance with an example of the present disclosure.

The method comprises a calibration procedure followed by a cataract surgical procedure. The calibration procedure is using the Bernoulli principle that correlates between the pressure and the flow rate of a fluid in a pumping system. More specifically, an increase in the speed of a fluid occurs simultaneously with a decrease in the static pressure of the fluid. The calibration procedure begins at a calibration chamber fitting step 100 with user of system 10 (e.g., physician 15 or a technician) receiving phacoemulsification probe 12 with a selected needle 16 having a tip with a predefined shape and diameter and a sleeve 56, as described in Fig. 1 above. In some examples, the user fits expandable calibration chamber 72 over needle 16 and sleeve 56.

In some examples, the inflation and deflation of calibration chamber 72 (as described in Fig. 2 above) are indicative of change in the static pressure of the fluids in calibration chamber 72. Note that during the calibration procedure, calibration chamber 72 resembles eye 20. Moreover, in addition to viewing the inflation and deflation, the user may use one or more sensors 74 (e.g., pressure and/or flow sensors) located in calibration chamber 72, phacoemulsification probe 12, anywhere along the irrigation and aspiration lines and/or in console 28, for sending to processor 38 signals indicative of the respective pressure and flow rate of the fluids flowing in system 10.

At an irrigation and aspiration step 102, processor 38 controls pump 44 to pump fluid into chamber 63, via irrigation line 43, at a constant target flow rate. Moreover, processor 38 records the irrigation flow rate reading sensed, during a predefined time interval (e.g., between about 10 seconds and 180 seconds), by the flow sensors described in step 100 above. While flowing the fluids through irrigation line 43, processor 38 controls pump 44 to pump fluid into aspiration chamber 66 via needle 16 and aspiration line 46, so as to obtain constant pressure in calibration chamber 72. Moreover, processor 38 receives from the sensors signals indicative of the measured pressure level in aspiration line 46, and records, during the same predefined time interval, the flow rate of the fluid flowing in aspiration line 46 and sensed by the flow sensor described in Fig. 1 above.

In alternative examples, step 102 may be carried out the other way around, i.e., flowing the aspiration fluid at a constant target flow rate, and controlling pump 44 to pump fluid via irrigation line 43, so as to obtain constant pressure in calibration chamber 72. In such examples, processor 38 receives from the sensors signals indicative of the measured pressure level in irrigation line 43 and/or in calibration chamber 72, and records, during the same predefined time interval, the flow rate of the fluid flowing in irrigation line 43 and sensed by the aforementioned flow sensors.

At a computation step 104, processor 38 is configured to compute integrals of the irrigation and aspiration flow rates readings recorded during the predefined time interval. Based on the computed integrals, processor 38 is configured to calculate a ratio between the irrigation and aspiration flow rates, which are suitable to obtain a constant pressure in calibration chamber 72. Note that the constant pressure in calibration chamber 72 is indicative of the pressure measured in irrigation line 43 and aspiration line 46. In other examples, pressure sensors may be positioned at the irrigation line 43 and aspiration line 46, and processor 38 may receive signals indicative of the sensed pressure.

In an example, in case the volume of aspiration chamber 66 is larger than that of irrigation chamber 63, e.g., by about 3%, based on the volume difference and the measured flow rate of the fluid in irrigation line 43, processor 38 is configured to calculate a ratio of about 1.03 between the aspiration and irrigation flow rates. In this example, for a measured irrigation flow rate of about 30 cc per minute, processor 38 is configured to set the target aspiration flow to a level of about 30.9, so as to compensate for the difference in the volumes, and to maintain the target pressure in the calibration chamber that resembles eye 20.

At a decision step 106, which is an optional step of the calibration procedure, the user decides whether he or she wants to select another cassette 55 or other operational conditions of system 10. For example, in case the user selects another cassette 55 (that may have different volumes of the irrigation and aspiration chambers), the method loops back to step 100 for calculating the flow rate ratio, as described in step 104 above. Note that in case the user has already selected the specific cassette and operational conditions that have already been used for calibrating system 10, then system 10 is ready to be used in the cataract surgical procedure.

In case the user does not want to select another cassette (instead of cassette 55) and/or other operational conditions, the method proceeds to a flow rate ratio storge step 108 that terminates the calibration procedure. In step 108, processor 38 stores one or more flow rate ratio(s) that define the irrigation/aspiration flow rate ratio for the respective combinations of a suitable type of cassette 55 and operational conditions. In alternative examples, processor 38 is configured to store one or more flow rate ratio(s) in a lookup table (LUT).

In some examples, after concluding the calibration procedure, system 10 is ready to carry out the cataract surgical procedure. In some examples, the surgical procedure may be carried out immediately after concluding the calibration procedure. In such embodiments, physician 15 removes calibration chamber 72 from phacoemulsification probe 12 and uses cassette 55 and the selected operational conditions of system 10 to carry out the cataract surgical procedure.

In other examples, the stored ratios calculated during the calibration procedure may be used in several cataract surgical procedures that may be carried out at any suitable time, when the same cassette 55 is being used for multiple procedures. Note that this combination of the predefined operational conditions and cassette 55 have been used together at the same time during the calibration procedure. Subsequently, physician 15 and/or processor 38 select (from among the stored flow rate ratios or LUT described in step 108 above) a suitable flow rate ratio to set flow rates of the irrigation and aspiration fluids. Note that the flow rates must be suitable under selected operational conditions of phacoemulsification probe 12, so as to obtain a predefined level of constant intraocular pressure in eye 20. After selecting the surgical tools (e.g., probe 12 and cassette 55), the operational conditions, the flow rate, and the flow rate ratio, physician 15 performs the phacoemulsification procedure as shown and described in Fig. 1 above.

The example flow chart shown in Fig. 3 is chosen purely for the sake of conceptual clarity. For example, additional steps, such controlling parameters of the piezoelectric crystal and other process parameters, are omitted for simplicity and clarity of presentation. Moreover, the flow rate ratios may be calculated using any suitable technique, other than the disclosed calibration procedure, and may be used by physician 15 to maintain constant IOP in eye 20 during the cataract surgical procedure.

Although the examples described herein mainly address phacoemulsification procedures. The methods and systems described herein can also be used in other applications.

As used herein, the terms "about" or "approximately" for any numerical values or ranges indicate a suitable dimensional tolerance that allows a physical dimension, such as a time interval, a physical part or collection of components, or a numerical value, such as a percentage or a ratio, to function for its intended purpose as described herein.

It will be appreciated that the examples described above are cited by way of example, and that the present disclosure is not limited to what has been particularly shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art.

## Claims

1. A phacoemulsification system, comprising:
a pump (44), comprising:
a first chamber (63)having a first volume and configured to flow a first fluid at a first flow rate, between a first reservoir and an eye (20) of a patient (19); and
a second chamber (66) having a second volume and configured to flow a second fluid at a second flow rate, between a second reservoir and the eye (20); and
a processor (38), configured to control the pump to:
set a first flow rate of the first fluid in the first volume, so as to obtain a predefined intra-ocular pressure, IOP, in the eye (20);
based on the first and second volumes and on the first flow rate, calculate a second flow rate of the second fluid that maintains the predefined IOP constant; and
set the calculated second flow rate to the second fluid, wherein the first volume is different from the second volume, and wherein the processor is configured to calculate the second flow rate to compensate for the difference between the first and second volumes.

2. The phacoemulsification system according to claim 1, wherein in a calibration step, the processor is configured to produce, for a given set of conditions, a dataset comprising: (i) a preset value of the first flow rate, and (ii) a calculated value of the second flow rate.

3. The phacoemulsification system according to claim 1 or claim 2, wherein the given set of conditions comprises: (i) the first and second volumes, and (ii) operational conditions of the phacoemulsification system.

4. The phacoemulsification system according to claim 1 or claim 2, wherein the first fluid comprises an irrigation fluid, which is applied to the eye at the first flow rate, and the second fluid comprises an aspiration fluid, which is aspired from the eye at the second flow rate.

5. The phacoemulsification system according to claim 1 or claim 2, wherein the first fluid comprises an aspiration fluid, which is aspired from the eye at the first flow rate, and the second fluid comprises an irrigation fluid, which is applied to the eye at the second flow rate.

6. The phacoemulsification system according to claim 1 or claim 2, wherein the pump comprises first and second progressive cavity pumps, PCPs, integrated together, optionally wherein the first PCP comprises the first chamber and a first electric motor, which is configured to flow the first fluid, and the second PCP comprises the second chamber and a second electric motor, which is configured to flow the second fluid.

7. The phacoemulsification system according to any of claims 1 to 6, further comprising one or more sensors, which are configured to sense at least one of: (i) the first flow rate, (ii) the second flow rate, (iii) a first pressure of the first fluid, and (iv) a second pressure of the second fluid.

8. A calibration method, comprising:
mounting an expandable calibration chamber (72) on a needle (16) and a sleeve (56)of a phacoemulsification probe (12), which is coupled with a pump (44);
activating the pump (44) to enable: (i) a first fluid at a first flow rate, to flow via a first tube (43) coupled between a first reservoir and the calibration chamber (72), and (ii) a second fluid at a second flow rate, to flow via a second tube coupled between a second reservoir and the calibration chamber (72);
setting a target value of the first flow rate, and while flowing the first fluid in the first tube (43), recording readings of the first flow rate over a predefined time interval, and concurrently: (i) flowing the second fluid in the second tube (46), and (ii) adjusting the second flow rate to maintain a constant pressure in the calibration chamber (72), and recording readings of the second flow rate over the predefined time interval; and
calculating a ratio between the readings of the first flow rate and the readings of the second flow rate.

9. The method according to claim 8, wherein the pump comprises a cassette having: (i) a first chamber having a first volume for flowing the first fluid, and (ii) a second chamber having a second volume for flowing the second fluid, and wherein when the first and second volumes differ from one another, the readings of the second flow rate differ from the readings of the first flow rate, optionally wherein the cassette has a first inner volume for flowing at least one of the first fluid and the second fluid, further comprising: (i) replacing the cassette with an additional cassette having a second inner volume, for flowing at least one of the first fluid and the second fluid, which is different from the first inner volume, (ii) activating the additional cassette to enable the first flow rate and the second flow rate, (iii) setting the target value and recording the readings, (iv) calculating an additional ratio between the readings of the first flow rate and the readings of the second flow rate, and (v) storing the ratio and the additional ratio in a lookup table.

10. The method according to claim 8, wherein calculating the ratio comprises computing, over the predefined time interval, (i) a first integral on the readings of the first flow rate, and (ii) a second integral on the readings of the second flow rate, and calculating the ratio between the first integral and the second integral.

11. The method according to claim 8, wherein the calibration chamber resembles an eye of a patient intended to undergo a phacoemulsification procedure using the phacoemulsification probe.

12. The method according to claim 10, wherein the first fluid comprises an irrigation fluid, which is intended to be applied to the eye at the first flow rate, and the second fluid comprises an aspiration fluid, which is intended to be aspired from the eye at the second flow rate, or
wherein the first fluid comprises an aspiration fluid, which is intended to be aspired from the eye at the first flow rate, and the second fluid comprises an irrigation fluid, which is intended to be applied to the eye at the second flow rate.

13. The method according to any of claims 8 to 12, further comprising storing the calculated ratio for use in a phacoemulsification procedure using the phacoemulsification probe.

14. The method according to claim 8, wherein the pump comprises a cassette having a first inner volume for flowing at least one of the first fluid and the second fluid, further comprising: (i) replacing the cassette with an additional cassette having a second inner volume, for flowing at least one of the first fluid and the second fluid, which is different from the first inner volume, (ii) activating the additional cassette to enable the first flow rate and the second flow rate, (iii) setting the target value and recording the readings, (iv) calculating an additional ratio between the readings of the first flow rate and the readings of the second flow rate, and (v) storing the ratio and the additional ratio in a lookup table.

15. The method according to claim 8, further comprising: (i) altering at least one of the first flow rate, and the pressure in the calibration chamber, (ii) calculating an additional ratio between the readings of the first flow rate and the readings of the second flow rate, and (iii) storing the ratio and the additional ratio in a lookup table.

## Patentansprüche

1. Phakoemulsifikationssystem, umfassend:
eine Pumpe (44), umfassend:
eine erste Kammer (63), die ein erstes Volumen aufweist und konfiguriert ist, um ein erstes Fluid mit einer ersten Strömungsgeschwindigkeit zwischen einem ersten Reservoir und einem Auge (20) eines Patienten (19) strömen zu lassen; und
eine zweite Kammer (66), die ein zweites Volumen aufweist und konfiguriert ist, um ein zweites Fluid mit einer zweiten Strömungsgeschwindigkeit zwischen einem zweiten Reservoir und dem Auge (20) strömen zu lassen; und einen Prozessor (38), der konfiguriert ist, um die Pumpe zu steuern zum:
Einstellen einer ersten Strömungsgeschwindigkeit des ersten Fluids in dem ersten Volumen, um einen vordefinierten Augeninnendruck, IOP, in dem Auge (20) zu erhalten;
basierend auf dem ersten und dem zweiten Volumen und auf der ersten Strömungsgeschwindigkeit, Berechnen einer zweiten Strömungsgeschwindigkeit des zweiten Fluids, die den vordefinierten IOP konstant hält; und
Einstellen der berechneten zweiten Strömungsgeschwindigkeit für das zweite Fluid, wobei sich das erste Volumen von dem zweiten Volumen unterscheidet, und wobei der Prozessor konfiguriert ist, um die zweite Strömungsgeschwindigkeit zu berechnen, um die Differenz zwischen dem ersten und dem zweiten Volumen auszugleichen.

2. Phakoemulsifikationssystem nach Anspruch 1, wobei in einem Kalibrierungsschritt der Prozessor konfiguriert ist, um für einen gegebenen Satz von Bedingungen einen Datensatz zu erzeugen, umfassend: (i) einen voreingestellten Wert der ersten Strömungsgeschwindigkeit und (ii) einen berechneten Wert der zweiten Strömungsgeschwindigkeit.

3. Phakoemulsifikationssystem nach Anspruch 1 oder 2, wobei der gegebene Satz von Bedingungen umfasst: (i) das erste und das zweite Volumen und (ii) Betriebsbedingungen des Phakoemulsifikationssystems.

4. Phakoemulsifikationssystem nach Anspruch 1 oder 2, wobei das erste Fluid ein Irrigationsfluid umfasst, das dem Auge mit der ersten Strömungsgeschwindigkeit zugeführt wird, und das zweite Fluid ein Aspirationsfluid umfasst, das mit der zweiten Strömungsgeschwindigkeit aus dem Auge aspiriert wird.

5. Phakoemulsifikationssystem nach Anspruch 1 oder 2, wobei das erste Fluid ein Aspirationsfluid umfasst, das mit der ersten Strömungsgeschwindigkeit aus dem Auge aspiriert wird, und das zweite Fluid ein Irrigationsfluid umfasst, das dem Auge mit der zweiten Strömungsgeschwindigkeit zugeführt wird.

6. Phakoemulsifikationssystem nach Anspruch 1 oder 2, wobei die Pumpe eine erste und eine zweite Exzenterschneckenpumpe, PCPs, umfasst, die zusammen integriert sind, gegebenenfalls wobei die erste PCP die erste Kammer und einen ersten elektrischen Motor umfasst, der konfiguriert ist, um das erste Fluid zu strömen zu lassen, und die zweite PCP die zweite Kammer und einen zweiten elektrischen Motor umfasst, der konfiguriert ist, um das zweite Fluid zu strömen zu lassen.

7. Phakoemulsifikationssystem nach einem der Ansprüche 1 bis 6, ferner umfassend einen oder mehrere Sensoren, die konfiguriert sind, um mindestens eines zu erfassen von: (i) der ersten Strömungsgeschwindigkeit, (ii) der zweiten Strömungsgeschwindigkeit, (iii) einem ersten Druck des ersten Fluids und (iv) einem zweiten Druck des zweiten Fluids.

8. Kalibrierungsverfahren, umfassend:
Anbringen einer expandierbaren Kalibrierkammer (72) auf einer Nadel (16) und einer Hülse (56) einer Phakoemulsifikationssonde (12), die mit einer Pumpe (44) gekoppelt ist;
Aktivieren der Pumpe (44), um zu ermöglichen: (i) einem ersten Fluid, mit einer ersten Strömungsgeschwindigkeit über einen ersten Schlauch (43) zu strömen, der zwischen ein erstes Reservoir und die Kalibrierkammer (72) gekoppelt ist, und (ii) einem zweiten Fluid, mit einer zweiten Strömungsgeschwindigkeit über einen zweiten Schlauch zu strömen, der zwischen ein zweites Reservoir und die Kalibrierkammer (72) gekoppelt ist;
Einstellen eines Zielwerts der ersten Strömungsgeschwindigkeit, und während des Strömenlassens des ersten Fluids in dem ersten Schlauch (43), Aufzeichnen von Messwerten der ersten Strömungsgeschwindigkeit über ein vordefiniertes Zeitintervall, und gleichzeitig: (i) Strömenlassen des zweiten Fluids in dem zweiten Schlauch (46) und (ii) Anpassen der zweiten Strömungsgeschwindigkeit, um einen konstanten Druck in der Kalibrierkammer (72) aufrechtzuerhalten, und Aufzeichnen von Messwerten der zweiten Strömungsgeschwindigkeit über das vordefinierte Zeitintervall; und
Berechnen eines Verhältnisses zwischen den Messwerten der ersten Strömungsgeschwindigkeit und den Messwerten der zweiten Strömungsgeschwindigkeit.

9. Verfahren nach Anspruch 8, wobei die Pumpe eine Kassette umfasst, die aufweist: (i) eine erste Kammer, die ein erstes Volumen zum Strömenlassen des ersten Fluids aufweist, und (ii) eine zweite Kammer, die ein zweites Volumen zum Strömenlassen des zweiten Fluids aufweist, und wobei, wenn sich das erste und das zweite Volumen voneinander unterscheiden, die Messwerte der zweiten Strömungsgeschwindigkeit sich von den Messwerten der ersten Strömungsgeschwindigkeit unterscheiden, gegebenenfalls wobei die Kassette ein erstes Innenvolumen zum Strömenlassen mindestens eines des ersten Fluids und des zweiten Fluids aufweist, ferner umfassend: (i) Ersetzen der Kassette durch eine zusätzliche Kassette, die ein zweites Innenvolumen aufweist, zum Strömenlassen mindestens eines des ersten Fluids und des zweiten Fluids, das sich von dem ersten Innenvolumen unterscheidet, (ii) Aktivieren der zusätzlichen Kassette, um die erste Strömungsgeschwindigkeit und die zweite Strömungsgeschwindigkeit zu ermöglichen, (iii) Einstellen des Zielwerts und Aufzeichnen der Messwerte, (iv) Berechnen eines zusätzlichen Verhältnisses zwischen den Messwerten der ersten Strömungsgeschwindigkeit und den Messwerten der zweiten Strömungsgeschwindigkeit, und (v) Speichern des Verhältnisses und des zusätzlichen Verhältnisses in einer Nachschlagetabelle.

10. Verfahren nach Anspruch 8, wobei das Berechnen des Verhältnisses das Berechnen, über das vordefinierte Zeitintervall, (i) eines ersten Integrals über die Messwerte der ersten Strömungsgeschwindigkeit und (ii) eines zweiten Integrals über die Messwerte der zweiten Strömungsgeschwindigkeit und das Berechnen des Verhältnisses zwischen dem ersten Integral und dem zweiten Integral umfasst.

11. Verfahren nach Anspruch 8, wobei die Kalibrierkammer einem Auge eines Patienten ähnelt, der bestimmt ist, sich einem Phakoemulsifikationseingriff unter Verwendung der Phakoemulsifikationssonde zu unterziehen.

12. Verfahren nach Anspruch 10,
wobei das erste Fluid ein Irrigationsfluid umfasst, das bestimmt ist, dem Auge mit der ersten Strömungsgeschwindigkeit zugeführt zu werden, das zweite Fluid ein Aspirationsfluid umfasst, das bestimmt ist, mit der zweiten Strömungsgeschwindigkeit aus dem Auge aspiriert zu werden, oder
wobei das erste Fluid ein Aspirationsfluid umfasst, das bestimmt ist, mit der ersten Strömungsgeschwindigkeit aus dem Auge aspiriert zu werden, und das zweite Fluid ein Irrigationsfluid umfasst, das bestimmt ist, mit der zweiten Strömungsgeschwindigkeit dem Auge zugeführt zu werden.

13. Verfahren nach einem der Ansprüche 8 bis 12, ferner umfassend das Speichern des berechneten Verhältnisses zur Verwendung in einem Phakoemulsifikationseingriff unter Verwendung der Phakoemulsifikationssonde.

14. Verfahren nach Anspruch 8, wobei die Pumpe eine Kassette umfasst, die ein erstes Innenvolumen zum Strömenlassen von mindestens einem von dem ersten Fluid und dem zweiten Fluid aufweist, ferner umfassend: (i) Ersetzen der Kassette durch eine zusätzliche Kassette, die ein zweites Innenvolumen aufweist, zum Strömenlassen mindestens eines des ersten Fluids und des zweiten Fluids, das sich von dem ersten Innenvolumen unterscheidet, (ii) Aktivieren der zusätzlichen Kassette, um die erste Strömungsgeschwindigkeit und die zweite Strömungsgeschwindigkeit zu ermöglichen, (iii) Einstellen des Zielwerts und Aufzeichnen der Messwerte, (iv) Berechnen eines zusätzlichen Verhältnisses zwischen den Messwerten der ersten Strömungsgeschwindigkeit und den Messwerten der zweiten Strömungsgeschwindigkeit, und (v) Speichern des Verhältnisses und des zusätzlichen Verhältnisses in einer Nachschlagetabelle.

15. Verfahren nach Anspruch 8, ferner umfassend: (i) Ändern mindestens eines von der ersten Strömungsgeschwindigkeit und dem Druck in der Kalibrierkammer, (ii) Berechnen eines zusätzlichen Verhältnisses zwischen den Messwerten der ersten Strömungsgeschwindigkeit und den Messwerten der zweiten Strömungsgeschwindigkeit, und (iii) Speichern des Verhältnisses und des zusätzlichen Verhältnisses in einer Nachschlagetabelle.

## Revendications

1. Système de phacoémulsification, comprenant :
une pompe (44), comprenant :
une première chambre (63) ayant un premier volume et conçue pour faire s'écouler un premier fluide à un premier débit, entre un premier réservoir et un œil (20) d'un patient (19) ; et
une seconde chambre (66) ayant un second volume et conçue pour faire s'écouler un second fluide à un second débit, entre un second réservoir et l'œil (20) ; et un processeur (38), configuré pour commander la pompe pour :
définir un premier débit du premier fluide dans le premier volume, de manière à obtenir une pression intraoculaire, PIO, prédéfinie dans l'œil (20) ;
sur la base des premier et second volumes et du premier débit, calculer un second débit du second fluide qui maintient la PIO prédéfinie constante ; et
définir le second débit calculé au second fluide, dans lequel le premier volume est différent du second volume, et dans lequel le processeur est configuré pour calculer le second débit pour compenser la différence entre les premier et second volumes.

2. Système de phacoémulsification selon la revendication 1, dans lequel, lors d'une étape d'étalonnage, le processeur est configuré pour produire, pour un ensemble donné de conditions, un ensemble de données comprenant : (i) une valeur prédéfinie du premier débit, et (ii) une valeur calculée du second débit.

3. Système de phacoémulsification selon la revendication 1 ou la revendication 2, dans lequel l'ensemble donné de conditions comprend : (i) les premier et second volumes, et (ii) des conditions de fonctionnement du système de phacoémulsification.

4. Système de phacoémulsification selon la revendication 1 ou la revendication 2, dans lequel le premier fluide comprend un fluide d'irrigation, qui est appliqué à l'œil au premier débit, et le second fluide comprend un fluide d'aspiration, qui est aspiré de l'œil au second débit.

5. Système de phacoémulsification selon la revendication 1 ou la revendication 2, dans lequel le premier fluide comprend un fluide d'aspiration, qui est aspiré de l'œil au premier débit, et le second fluide comprend un fluide d'irrigation, qui est appliqué à l'œil au second débit.

6. Système de phacoémulsification selon la revendication 1 ou la revendication 2, dans lequel la pompe comprend de première et seconde pompes à vis hélicoïdale excentrée, PCP, intégrées ensemble, éventuellement dans lequel la première PCP comprend la première chambre et un premier moteur électrique, qui est configuré pour faire s'écouler le premier fluide, et la seconde PCP comprend la seconde chambre et un second moteur électrique, qui est configuré pour faire s'écouler le second fluide.

7. Système de phacoémulsification selon l'une quelconque des revendications 1 à 6, comprenant en outre un ou plusieurs capteurs, qui sont configurés pour détecter au moins l'un parmi : (i) le premier débit, (ii) le second débit, (iii) une première pression du premier fluide et (iv) une seconde pression du second fluide.

8. Procédé d'étalonnage, comprenant :
le montage d'une chambre d'étalonnage expansible (72) sur une aiguille (16) et un manchon (56) d'une sonde de phacoémulsification (12), qui est accouplée à une pompe (44) ;
l'activation de la pompe (44) pour permettre : (i) l'écoulement d'un premier fluide à un premier débit, par l'intermédiaire d'un premier tube (43) accouplé entre un premier réservoir et la chambre d'étalonnage (72), et (ii) l'écoulement d'un second fluide à un second débit, par l'intermédiaire d'un second tube accouplé entre un second réservoir et la chambre d'étalonnage (72) ;
la définition d'une valeur cible du premier débit, et tout en faisant s'écouler le premier fluide dans le premier tube (43), l'enregistrement de relevés du premier débit sur un intervalle de temps prédéfini, et simultanément : (i) l'écoulement du second fluide dans le second tube (46), et (ii) l'ajustement du second débit pour maintenir une pression constante dans la chambre d'étalonnage (72), et l'enregistrement des relevés du second débit sur l'intervalle de temps prédéfini ; et
le calcul d'un rapport entre les relevés du premier débit et les relevés du second débit.

9. Procédé selon la revendication 8, dans lequel la pompe comprend une cassette ayant : (i) une première chambre ayant un premier volume pour l'écoulement du premier fluide, et (ii) une seconde chambre ayant un second volume pour l'écoulement du second fluide, et dans lequel, lorsque les premier et second volumes diffèrent l'un de l'autre, les relevés du second débit diffèrent des relevés du premier débit, éventuellement dans lequel la cassette a un premier volume interne pour l'écoulement d'au moins l'un parmi le premier fluide et le second fluide, comprenant en outre : (i) le remplacement de la cassette par une cassette supplémentaire ayant un second volume interne, pour l'écoulement d'au moins l'un parmi le premier fluide et le second fluide, qui est différent du premier volume interne, (ii) l'activation de la cassette supplémentaire pour permettre le premier débit et le second débit, (iii) la définition de la valeur cible et l'enregistrement des relevés, (iv) le calcul d'un rapport supplémentaire entre les relevés du premier débit et les relevés du second débit, et (v) le stockage du rapport et du rapport supplémentaire dans une table de consultation.

10. Procédé selon la revendication 8, dans lequel le calcul du rapport comprend le calcul informatisé, sur l'intervalle de temps prédéfini, (i) d'une première intégrale sur les relevés du premier débit, et (ii) d'une seconde intégrale sur les relevés du second débit, et le calcul du rapport entre la première intégrale et la seconde intégrale.

11. Procédé selon la revendication 8, dans lequel la chambre d'étalonnage ressemble à un œil d'un patient destiné à subir une procédure de phacoémulsification à l'aide de la sonde de phacoémulsification.

12. Procédé selon la revendication 10,
dans lequel le premier fluide comprend un fluide d'irrigation, qui est destiné à être appliqué à l'œil au premier débit, et le second fluide comprend un fluide d'aspiration, qui est destiné à être aspiré de l'œil au second débit, ou
dans lequel le premier fluide comprend un fluide d'aspiration, qui est destiné à être aspiré de l'œil au premier débit, et le second fluide comprend un fluide d'irrigation, qui est destiné à être appliqué à l'œil au second débit.

13. Procédé selon l'une quelconque des revendications 8 à 12, comprenant en outre le stockage du rapport calculé pour une utilisation dans une procédure de phacoémulsification à l'aide de la sonde de phacoémulsification.

14. Procédé selon la revendication 8, dans lequel la pompe comprend une cassette ayant un premier volume interne pour l'écoulement d'au moins l'un parmi le premier fluide et le second fluide, comprenant en outre : (i) le remplacement de la cassette par une cassette supplémentaire ayant un second volume interne, pour l'écoulement d'au moins l'un parmi le premier fluide et le second fluide, qui est différent du premier volume interne, (ii) l'activation de la cassette supplémentaire pour permettre le premier débit et le second débit, (iii) la définition de la valeur cible et l'enregistrement des relevés, (iv) le calcul d'un rapport supplémentaire entre les relevés du premier débit et les relevés du second débit, et (v) le stockage du rapport et du rapport supplémentaire dans une table de consultation.

15. Procédé selon la revendication 8, comprenant en outre : (i) la modification d'au moins l'un parmi le premier débit et la pression dans la chambre d'étalonnage, (ii) le calcul d'un rapport supplémentaire entre les relevés du premier débit et les relevés du second débit, et (iii) le stockage du rapport et du rapport supplémentaire dans une table de consultation.
